# EUROPÄISCHE PATENTSCHRIFT

(11) **EP 1 935 350 B1**
(45) Veröffentlichungstag und Bekanntmachung des Hinweises auf die Patenterteilung: **05.12.2012**
(21) Anmeldenummer: 07024699.6
(22) Anmeldetag: 20.12.2007
(51) Int. Cl.: A61B 17/04

(54) **Vorrichtung zum Einbringen eines Ankerelements in einen Knochen**
Device for attaching an anchor element to a bone
Dispositif destiné à introduire un élément d'ancrage dans un os

(30) Priorität: 21.12.2006 DE 102006062401
(43) Veröffentlichungstag der Anmeldung: 25.06.2008
(73) Patentinhaber: Karl Storz GmbH & Co. KG, 78532 Tuttlingen (DE)
(72) Erfinder: Berberich, Sascha, 78532 Tuttlingen (DE)
(74) Vertreter: Weller, Wolfgang

(56) Entgegenhaltungen:
- EP-A- 1 719 450
- WO-A-98/52471
- WO-A-2004/062507
- US-A- 5 690 676
- US-A- 5 868 789

## Beschreibung

Die Erfindung betrifft eine Vorrichtung zum Einbringen eines Ankerelements, in dem ein Faden eingefädelt ist, in einen Knochen zum Fixieren eines vom Knochen abgelösten Gewebes, mit einem langerstreckten Körper, dessen distales Ende derart ausgebildet ist, dass es mit dem Ankerelement drehschlüssig verbindbar ist, wobei ein distaler Endabschnitt des Körpers mit zumindest einer Aussparung versehen ist, in der eine Schlaufe des Fadens einlegbar ist, wobei die Aussparung derart ausgebildet ist, dass die eingelegte Schlaufe vor seitlichem Abfallen gehindert ist, und wobei im Bereich der Aussparung in dem Körper eine Ausnehmung vorhanden ist, die die Aussparung schneidet.

Eine solche Vorrichtung zum Einbringen eines Ankerelements mit diesen konstruktiven Merkmalen ist aus der WO 98/52471 bekannt. WO 98/52471 offenbart die Merkmale des Preambels des Anspruchs 1.

Eine weitere ähnliche Vorrichtung ist aus dem Firmenkatalog der Firma Karl Storz GmbH & Co. KG, Tuttlingen, "STORZ, DIE WELT DER ENDOSKOPIE; ARTHROSKOPIE, SPORTMEDIZIN, WIRBELSÄULEN-CHIRURGIE", 2. Ausgabe 1/2005, Seite 114 (Geräte-Nr. 28179 TI), bekannt.

Derartige Vorrichtungen dienen zum Einbringen eines Ankerelements, in dem ein Faden eingefädelt ist, in einen Knochen, so dass das Ankerelement in dem Knochen verankert wird. Beide Fadenenden des in dem Ankerelement eingefädelten Fadens werden mit einer abgerissenen Sehne oder einem abgerissenen Band verknotet, und diese(r) wird dadurch wieder an dem Knochen fixiert. Ein Haupteinsatzgebiet für solche Ankerelemente ist das Fixieren von abgerissenen Sehnen im Schulterbereich.

Die genannte Vorrichtung der WO 98/52471 weist einen langerstreckten Körper auf, an dessen proximalem Ende ein Griff angeordnet ist. Ein distales Ende des Körpers ist mit einer beispielsweise schlitzförmigen Ausnehmung versehen, in die ein Vorsprung am proximalen Ende des Ankerelements einbringbar ist. Am proximalen Ende weist der langerstreckte Körper eine Klemmvorrichtung für den durch das Ankerelement durchgefädelten Faden auf. Dieser verläuft vom distal gelegenen Ankerelement auf gegenüberliegenden Seiten des langerstreckten Körpers von distal nach proximal und wird zum Zwecke einer besseren Stabilisierung und Fixierung des Ankerelements an der Vorrichtung an einer am distalen Ende des langerstreckten Körpers befindlichen Aussparung über Kreuz geführt.

Die aus dem Storz-Katalog genannte Vorrichtung weist ebenfalls einen langerstreckten Körper auf, der ebenfalls an seinem proximalen Ende einen Griff aufweist. Ein distales Ende des Körpers ist mit einem Vorsprung versehen, der in eine Öffnung in dem Ankerelement einbringbar ist. Der distale Vorsprung ist derart ausgebildet, dass die Vorrichtung mit dem Ankerelement drehschlüssig verbindbar ist. Ferner weist die aus dem Storz-Katalog bekannte Vorrichtung ebenfalls eine seitliche Klemmvorrichtung für den durch das Ankerelement durchgefädelten Faden auf. Für den Eintreibevorgang des Ankerelements in den Knochen werden die beiden Enden des durch das Ankerelement durchgefädelten Fadens längs des Körpers geführt und dann um die radial vorstehende Klemmvorrichtung gewickelt.

Das Einbringen des Ankerelements in den Knochen mittels der oben genannten Vorrichtungen kann auf zwei Vorgehensweisen erfolgen, abhängig davon, welches Ankerelement verwendet wird.

Bei der ersten Vorgehensweise wird ein Kanal in dem Knochen gebohrt, und in diesen vorgebohrten Kanal wird ein Ankerelement eingebracht. Dazu wird das Ankerelement auf den distalen Vorsprung der Vorrichtung aufgesetzt und in die Bohrung eingedreht. Nachdem das Ankerelement gesetzt ist, wird die Vorrichtung abgenommen, und die Sehne wird mit den Fadenenden an dem Knochen fixiert. Damit das Ankerelement sich in der Bohrung verankert, sind an seiner Außenseite hakenartige oder widerhakenartige Vorsprünge vorhanden.

Bei einer zweiten Vorgehensweise reicht die Spitze des Vorsprungs der Vorrichtung über das distale Ende des Ankerelements hinaus, an dessen Außenseite ein Gewinde vorgesehen ist. Durch ein Einschlagen gefolgt von einer Drehbewegung der mit dem Ankerelement drehschlüssig verbundenen Vorrichtung kann sich das Ankerelement in den Knochen einfressen und dadurch verankert werden. Somit kann ein derartig ausgebildetes Ankerelement ohne vorheriges Bewerkstelligen einer Bohrung eingesetzt werden.

Unabhängig von der Art des verwendeten Ankerelements werden die Enden des durch das Ankerelement durchgefädelten Fadens, die während des Einbringens des Ankerelements in den Knochen an der seitlichen Klemmvorrichtung aufgewickelt sind, nachdem das Ankerelement in den Knochen eingesetzt ist, von der Klemmvorrichtung gelöst. Bevor mit den beiden Enden des Fadens die abgerissene Sehne verknotet wird, müssen die zwei Fadenenden eines nach dem anderen arthroskopisch separiert werden, was äußerst kompliziert ist und selbst von einem erfahrenen Operateur sehr viel Geschicklichkeit erfordert.

Es wurde nunmehr eine Operationstechnik entwickelt, bei der der Faden von einer Seite her durch die Queröffnung im Anker hindurchgeführt wird, auf der anderen Seite zu einer Schlaufe gebogen wird und wieder in umgekehrter Richtung durch die Querbohrung zurückgeführt wird.

Somit steht von einer Seite des Ankerelementes seitlich eine Schlaufe des Fadens vor, auf der gegenüberliegenden Seite zwei freie Fadenenden.

Bei Einsatz der eingangs genannten Vorrichtung aus dem Storz-Katalog mit einer solchen Fadenführung im Ankerelement traten Schwierigkeiten mit der Fixierung der Schlaufe an der Vorrichtung für das Eindrehen auf.

Aus der WO 2004/062507 A2 ist eine Vorrichtung zum Einbringen eines Ankerelementes, in dem ein Faden eingefädelt ist, in einen Knochen bekannt. Ein distales Ende der Vorrichtung ist derart ausgebildet, dass es mit dem Ankerelement drehschlüssig verbindbar ist. Dazu kann das distale Ende in das Ankerelement eingeschoben werden. In dem distalen Ende ist eine Aussparung vorgesehen, in die eine Schlaufe des im Inneren des Ankerelements aufgenommenen Fadens eingehängt werden kann. Ist die Vorrichtung zum Einbringen mit dem Ankerelement verbunden, befindet sich die Schlaufe innerhalb des Ankerelements.

Aus der EP 1 484 022 A2 ist eine Vorrichtung zum Einbringen von zumindest einem Ankerelement, in dem ein Faden eingefädelt ist, in einen Knochen bekannt. Das Ankerelement weist seitlich vorstehende Flügel auf. Dazu ist im Körper der Vorrichtung zum Einbringen des Ankerelements ein seitlicher Schlitz vorgesehen, über den die Flügel seitlich vorstehen können. In einer Variante ist vorgesehen, mehrere Ankerelemente in die Vorrichtung einzubringen. Da durch die Ankerelemente Fäden durchgeführt sind, ist zusätzlich eine weitere seitliche Öffnung in dem Körper der Vorrichtung zum Einbringen vorgesehen, über die die Fäden seitlich herausgeführt werden können.

Der Erfindung liegt daher die Aufgabe zugrunde, eine Vorrichtung zum Einbringen eines Ankerelements in einen Knochen der eingangs genannten Art dahingehend weiterzuentwickeln, dass bei einfacher Bauweise der Faden während des Einbringens sicher gehalten ist und anschließend einfach separiert werden kann.

Erfindungsgemäß wird die Aufgabe dadurch gelöst, dass die Ausnehmung sich in Längsrichtung des Körpers erstreckt und als durch den Körper hindurchgehende Öffnung ausgebildet ist, und dass die Aussparung als ein sich quer zur Öffnung erstreckender Einschnitt ausgebildet ist, so dass ein Fadenabschnitt, der in den Einschnitt eingelegt ist, freiliegend die Ausnehmung überquert und daher von der Außenseite ergreifbar ist.

Diese Maßnahme hat den Vorteil, dass die Schlaufe nach Ansetzen der Vorrichtung an das Ankerelement in die Aussparung eingelegt werden kann. Durch Ziehen an den freien Enden wird die Schlaufe straff sitzend in die Aussparung eingefädelt. Nach Befestigung der freien Enden an der Klemmvorrichtung ist die Schlaufe unverlierbar an der Vorrichtung gehalten.

Dann wird das Ankerelement mittels der erfindungsgemäßen Vorrichtung auf eine der oben beschriebenen Vorgehensweisen in den Knochen eingebracht. Nachdem das Ankerelement in den Knochen gesetzt worden ist und die freien Enden von der Klemmvorrichtung gelöst wurden, kann die Schlaufe des Fadens wieder aus der Aussparung herausbewegt werden. Durch das Einlegen der Schlaufe des Fadens in der an dem distalen Endabschnitt ausgebildeten Aussparung wird die Schlaufe in eine vorbestimmte Position gebracht, die für den Operateur ersichtlich ist. Daher kann die Schlaufe von dem Operateur gezielt ergriffen werden, da er die genaue Position der Schlaufe kennt.

Dadurch, dass ein Fadenabschnitt der in der Aussparung eingelegten Schlaufe im Bereich der Ausnehmung freiliegend ist, kann die Schlaufe sehr einfach von dem Operateur ergriffen werden.

Dadurch, dass sich die Ausnehmung in Längsrichtung des Körpers erstreckt, also sowohl quer zu der als Einschnitt ausgebildeten Aussparung als auch zu dem Fadenabschnitt der in der Aussparung eingelegten Schlaufe, kann der Fadenabschnitt von der Außenseite her einfach mit einem Instrument, dessen distales Ende beispielsweise hakenartig ausgebildet ist, ergriffen werden.

Diese Ausgestaltung der Ausnehmung in Form einer hindurchgehenden Öffnung hat den Vorteil, dass die Schlaufe von zwei Seiten her besonders einfach ergreifbar ist. Ein Instrument zum Ergreifen der Schlaufe kann zunächst durch die Ausnehmung hindurchgeführt und der Haken hinter die Schlaufe gebracht werden, um diese dann nach vorne abzuziehen.

In einer weiteren Ausgestaltung der Erfindung ist die Aussparung als ein sich quer zu einer Längsachse des Körpers erstreckender Einschnitt ausgebildet.

Diese Maßnahme hat den Vorteil, dass die in dem derart ausgebildeten Einschnitt eingelegte Schlaufe des Fadens durch ein Festziehen der freien Fadenenden radial in den Einschnitt hineingleitet und dadurch in dem Einschnitt fest sitzt. Somit ist die Schlaufe des gespannten Fadens während des Einbringens des Ankerelements in den Knochen vor seitlichem Abfallen gehindert.

In einer weiteren Ausgestaltung verläuft die Aussparung, von proximal nach distal gesehen, nach distal geneigt.

Durch diesen schrägen, geneigten Verlauf wird die Schlaufe radial nach innen und nach distal geführt und ist besonders sicher vor seitlichem Abfallen von der Vorrichtung gehindert.

In einer weiteren Ausgestaltung der Erfindung beträgt die Neigung 30° bis etwa 90°, vorzugsweise etwa 45°.

Durch eine ausreichend steile Neigung der Aussparung wird erreicht, dass die in der Aussparung eingelegte Schlaufe des Fadens selbst bei einer gelockerten Fadenspannung vor seitlichem Abfallen gehindert ist.

In einer weiteren Ausgestaltung der Erfindung erstreckt sich die Aussparung umfänglich, vorzugsweise halbumfänglich, um den Körper.

Diese Maßnahmen haben den Vorteil, dass eine derartig ausgebildete Aussparung der eingelegten Schlaufe des Fadens eine entsprechend große Anlagefläche zur Verfügung stellt. Dies trägt zu einer sicheren Fadenaufnahme, selbst bei einer gelockerten Fadenspannung, bei.

In einer weiteren Ausgestaltung der Erfindung weist die Aussparung in radialer Richtung gesehen eine Tiefe auf, die maximal der Hälfte des Durchmessers des Körpers entspricht.

Durch eine derartige Ausgestaltung der erfindungsgemäßen Vorrichtung wird durch das radial tiefe Einlegen zum einen erreicht, dass die Schlaufe des Fadens in der Aussparung besonders sicher vor seitlichem Abfallen gehindert ist. Zum anderen erlaubt eine derartige Ausbildung der Aussparung, dass auch bei geringem Durchmesser die Vorrichtung die notwendige Stabilität aufweist, um das Ankerelement in den Knochen einzubringen, was mit relativ hoher Kraftausübung auf die Vorrichtung verbunden ist.

In einer weiteren Ausgestaltung der Erfindung ist die Aussparung als Schlitz ausgebildet.

Diese Maßnahme hat den Vorteil, dass der Schlitz einfach herstellbar ist.

In einer weiteren Ausgestaltungen der Erfindung schneiden sich die Ausnehmung und die Aussparung mittig.

Diese Maßnahme hat den Vorteil, dass der Fadenabschnitt der in der Aussparung eingelegten Schlaufe mit einem Instrument nicht nur von zwei Seiten her ergriffen werden kann, sondern auch von oben und von unten.

Es versteht sich, dass die vorstehend genannten und die nachstehend noch zu erläuternden Merkmale nicht nur in der angegebenen Kombination, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels in Zusammenhang mit den beiliegenden Zeichnungen näher beschrieben und erläutert.
Es zeigen:
- Figur 1: eine perspektivische Seitenansicht einer erfindungsgemäßen Vorrichtung;
- Figur 2: eine perspektivische vergrößerte Seitenansicht eines distalen Endabschnitts der erfindungsgemäßen Vorrichtung von Figur 1;
- Figur 3: eine Seitenansicht des distalen Endabschnitts der Vorrichtung von Figur 2, wobei die Vorrichtung um 90° um eine Längsachse der Vorrichtung verdreht ist, sowie ein Ankerelement, durch das ein Faden mit einer Schlaufe durchgefädelt ist, wobei das Ankerelement mit der Vorrichtung noch nicht verbunden ist;
- Figur 4: eine Seitenansicht eines distalen Endabschnitts eines Zusammenbaus bestehend aus dem Ankerelement und der Vorrichtung von Figur 3, wobei eine Schlaufe des durch das Ankerelement durchgefädelten Fadens in einer Aussparung der Vorrichtung eingelegt ist;
- Figur 5: eine der Darstellung von Figur 4 vergleichbare, jedoch um 90° um die Längsachse der Vorrichtung verdrehte Darstellung;
- Figur 6: eine Situation beim Einbringen des Ankerelements in einen Schulterknochen mit der erfindungsgemäßen Vorrichtung, wobei der in Figur 5 dargestellte Zusammenbau ersichtlich ist; und
- Figur 7: eine der Darstellung von Figur 6 vergleichbare, jedoch um 90° verdrehte Darstellung, wobei die Schlaufe des durch das Ankerelement durchgefädelten Fadens von einem Instrument ergriffen ist.

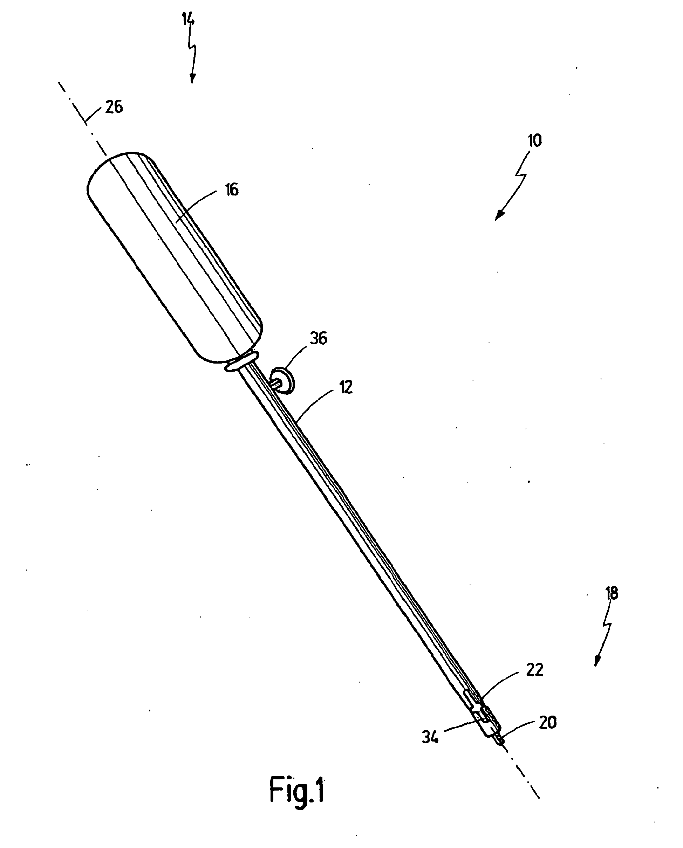

Eine in Figuren 1 bis 3 dargestellte Vorrichtung zum Einbringen eines Ankerelements in einen Knochen ist in ihrer Gesamtheit mit der Bezugsziffer 10 bezeichnet.

Die erfindungsgemäße Vorrichtung 10 weist einen langerstreckten Körper 12 auf, der vorzugsweise aus Metall hergestellt ist. An einem proximalen Ende 14 des Körpers 12 ist ein Griff 16 angeordnet.

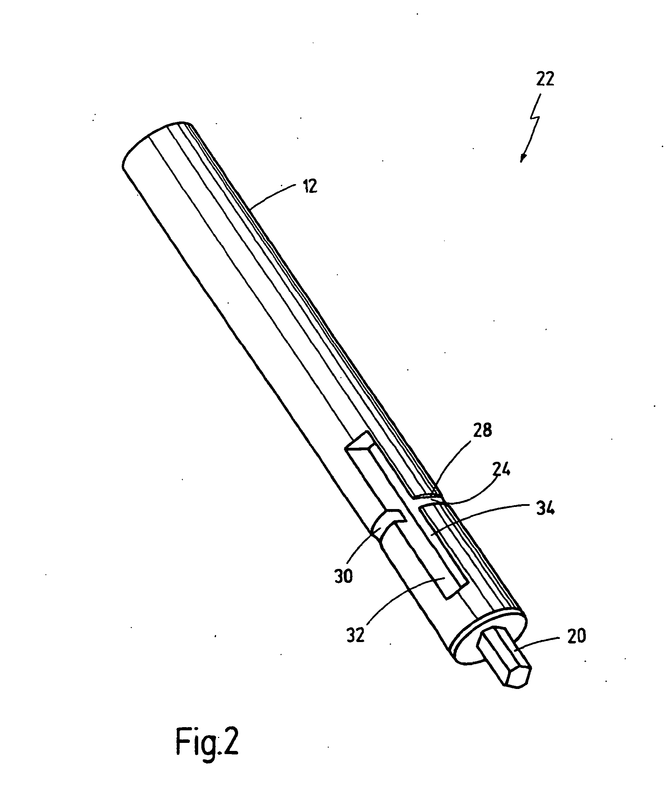

Ein distales Ende 18 des Körpers 12 ist mit einem Vorsprung 20 versehen, wie es insbesondere aus der vergrößerten Darstellung von Figur 2 hervorgeht. Auf den Vorsprung 20 ist ein hier nicht dargestelltes Ankerelement derart bringbar, dass das Ankerelement und die Vorrichtung 10 drehschlüssig verbunden werden. Bei diesem Ausführungsbeispiel ist der Vorsprung 20 im Querschnitt sechseckig ausgebildet. Er kann jedoch einen anderen Querschnitt aufweisen, abhängig davon, wie eine Öffnung in dem Ankerelement, mit dem die Vorrichtung 10 drehschlüssig verbunden werden soll, ausgebildet ist.

Ein distaler Endabschnitt 22 des Körpers 12 ist mit einer Aussparung 24 versehen.

Die Aussparung 24 ist als ein sich quer zu einer Längsachse 26 des Körpers 12 erstreckender Einschnitt 28 ausgebildet. Die Aussparung 24 erstreckt sich umfänglich um den Körper 12. Bei diesem in Figur 2 dargestellten Ausführungsbeispiel erstreckt sie sich halbumfänglich.

Die Aussparung 24 ist als Schlitz 30 ausgebildet, der in dem aus Metall bestehenden Körper 12 durch ein Ausfräsen einfach herstellbar ist.

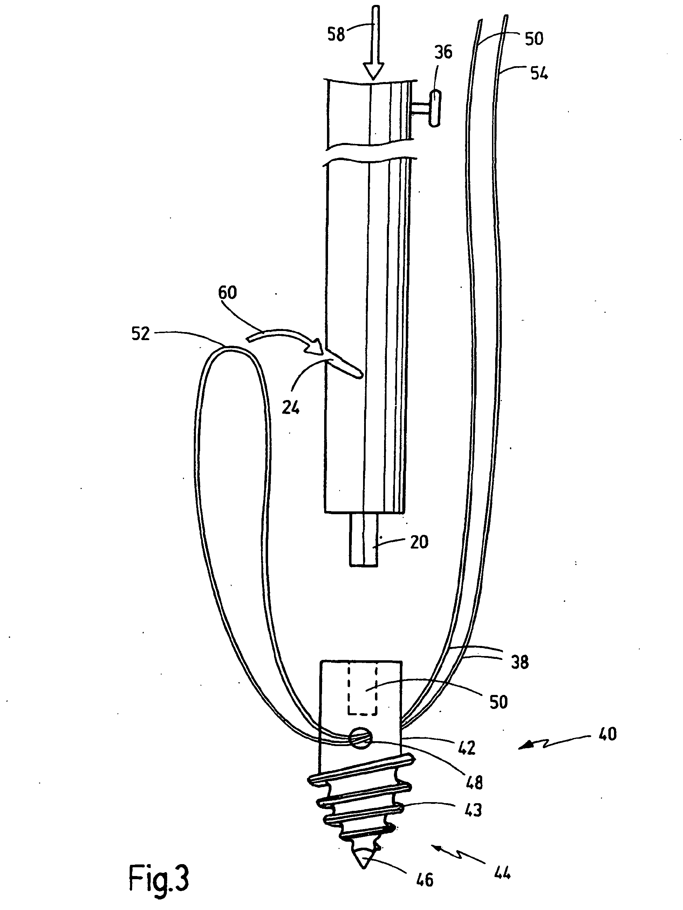

Die als Schlitz 30 ausgebildete Aussparung 24 weist in radialer Richtung gesehen eine Tiefe auf, die maximal der Hälfte des Durchmessers des Körpers 12 entspricht, wie es aus der Darstellung von Figur 2 und insbesondere von Figur 3 hervorgeht.

Die Aussparung 24 ist, von proximal nach distal gesehen, aus einer Querschnittsfläche nach distal geneigt, beispielsweise im Bereich von 30° bis etwa 90°. Bei dem gezeigten Ausführungsbeispiel weist die Aussparung 24 eine Neigung von 45° auf, wie es aus der Darstellung von Figur 3 ersichtlich ist.

Zusätzlich ist in dem distalen Endabschnitt 22 eine Ausnehmung 32 vorhanden, wie es aus der vergrößerten Darstellung von Figur 2 hervorgeht. Die Ausnehmung 32 erstreckt sich in Längsrichtung des Körpers 12 und ist als längsverlaufende Nut ausgebildet. Die Ausnehmung 32 ist als eine durch den Körper 12 hindurchgehende Öffnung 34 ausgebildet.

In Figur 3 ist eine Seitenansicht des distalen Endabschnitts 22 der erfindungsgemäßen Vorrichtung 10 dargestellt, wobei diese Ansicht im Vergleich zu der Darstellung von Figur 2 um 90° um die Längsachse 26 verdreht ist.

In dieser Ansicht ist ein Zapfen 36 ersichtlich, der von dem Körper 12 radial vorsteht. Der Zapfen 36 dient zum Fixieren der freien Enden 54, 56 eines Fadens 38 während des Einbringens eines Ankerelements 40 in den Knochen.

In Figur 3 ist ferner ein Ankerelement 40 dargestellt, das auf den Vorsprung 20 der erfindungsgemäßen Vorrichtung 10 aufsteckbar ist.

Das Ankerelement 40 weist einen Körper 42 auf, der sich distalseitig verjüngt. Mittig in dem Körper 42 ist von proximal eine axiale Vertiefung 50 eingebracht, die zur Aufnahme des distalen Vorsprungs 20 der Vorrichtung 10 dient. Die axiale Vertiefung 50 weist einen sechseckigen Querschnitt auf, der dem sechseckig ausgebildeten Vorsprung 20 entspricht.

Eine Außenseite des Körpers 42 ist mit einem Gewinde 43 versehen, das sich etwa von der Mitte des Körpers 42 bis zu einem distalen Ende 44 des Körpers 42 erstreckt. Das distale Ende 44 ist bei diesem Ausführungsbeispiel als Spitze 46 ausgebildet.

Im mittigen Bereich des Körpers 42 des Ankerelements 40 ist eine durch diesen hindurchgehende Querbohrung 48 angeordnet, die zum Durchfädeln des Fadens 38 durch den Körper 42 des Ankerelements 40 dient, wie es in Figur 3 dargestellt ist. Der Faden 38 ist durch die Querbohrung 48 doppelt durchgefädelt, so dass an einer Seite der Querbohrung 48 eine Schlaufe 52 des Fadens 38 vorsteht, an der anderen Seite der Querbohrung 48 dagegen zwei Fadenenden 54, 56 des Fadens 38 vorstehen.

Bevor das Ankerelement 40 in einen Knochen eingebracht wird, wird die Vorrichtung 10 mit dem Ankerelement 40 verbunden, indem der Vorsprung 20 der Vorrichtung 10 in die axiale Vertiefung 50 in dem Ankerelement 40 eingeführt wird. Die Richtung der Einführung ist durch einen Pfeil 58 angezeigt.

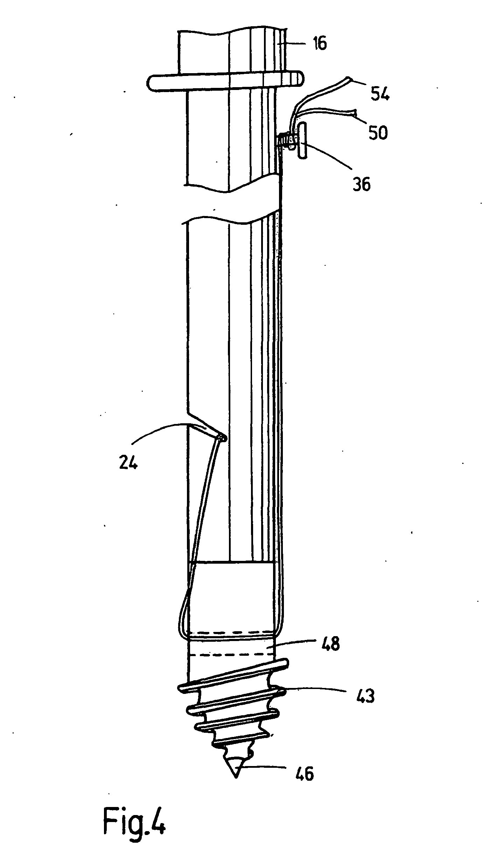

Dann wird die Schlaufe 52 des durch die Querbohrung 48 durchgefädelten Fadens 38 in die als Schlitz 30 ausgebildete Aussparung 24 eingelegt, wie dies durch einen Pfeil 60 angedeutet ist. Dann wird an den Fadenenden 54, 56 gezogen, so dass die Schlaufe straff gezogen im Schlitz 30 gehalten ist, und anschließend werden die Fadenenden 54, 56 auf den Zapfen 36 aufgewickelt, so wie es in Figur 4 dargestellt ist.

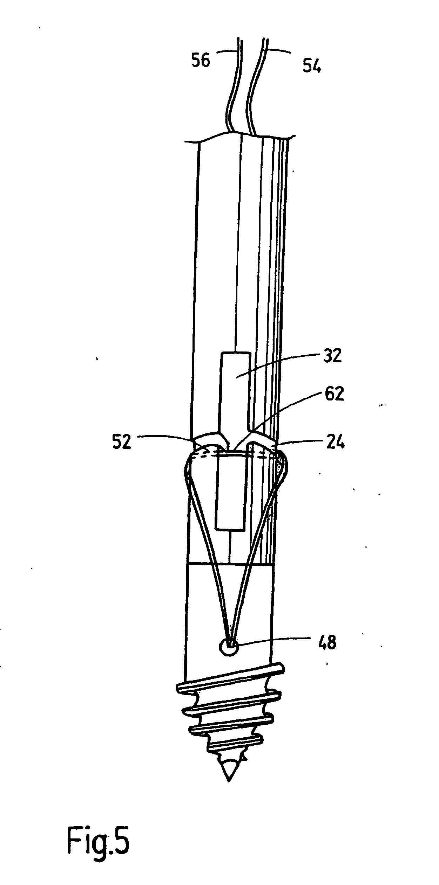

In Figur 5 ist der Zusammenbau bestehend aus dem Ankerelement 40 und der Vorrichtung 10 dargestellt, wobei der Zusammenbau im Vergleich zu der Darstellung von Figur 4 um 90° um die Längsachse 26 verdreht ist.

Wie aus dieser Darstellung ersichtlich ist, schneiden sich die Ausnehmung 32 und die Aussparung 24 mittig. Durch eine derartige Ausgestaltung der erfindungsgemäßen Vorrichtung liegt ein Fadenabschnitt 62 der in der Aussparung 24 eingelegten Schlaufe 52 des durch das Ankerelement 40 durchgefädelten Fadens 38 frei und ist einfach von der Außenseite her ergreifbar. Dadurch, dass sich die Ausnehmung 32 und die Aussparung 24 mittig schneiden, kann der Fadenabschnitt 64 von oben oder von unten her ergriffen werden.

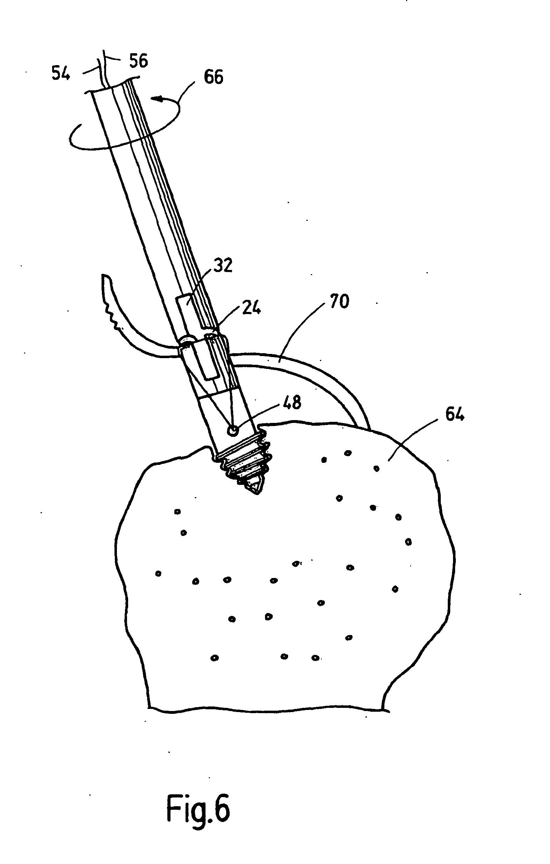

Zum Einbringen des Ankerelements 40 in einen Knochen wird der Zusammenbau von Figur 4 bzw. 5 z.B. in einen Schulterknochen 64 eingetrieben. Dazu wird durch Drehen des Zusammenbaus, wie das in Figur 6 durch einen Pfeil 66 dargestellt ist, der Zusammenbau in den Schulterknochen 64 eingedreht, wobei dies durch das Gewinde 43 unterstützt wird.

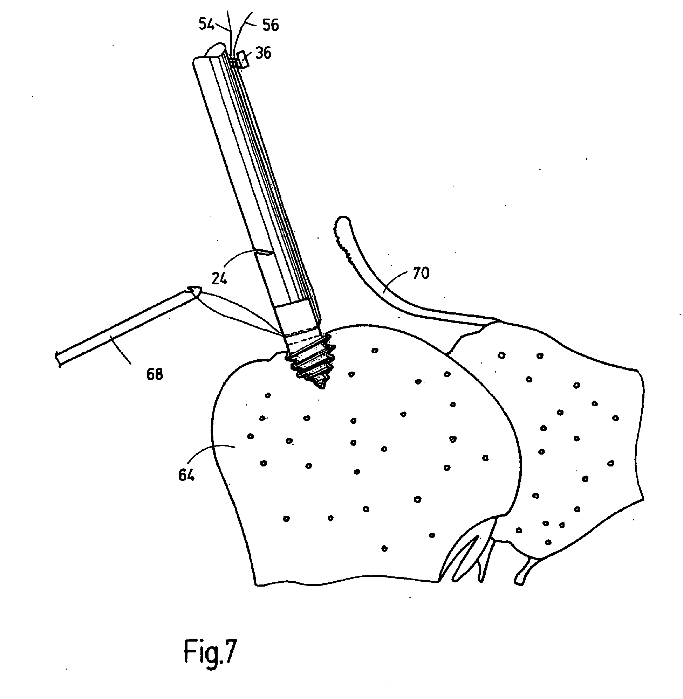

Nach dem vollständigen Eindrehen des Ankerelements 40 in den Schulterknochen 64 wird der Fadenabschnitt 62 der in der Aussparung 24 eingelegten Schlaufe 52 mit einem Instrument 68 ergriffen, wie es schematisch in Figur 7 dargestellt ist. Das Instrument 68 kann vorzugsweise ein hakenartig ausgebildetes distales Ende aufweisen, das in die Ausnehmung 32 der Vorrichtung 10 eingeführt werden kann.

Nachdem der Fadenabschnitt 62 durch das Instrument 68 gefangen worden ist, werden die Fadenenden 54, 56 von dem Zapfen 36 gelöst, und das Instrument 68 wird mit der Schlaufe 52 von der Vorrichtung abgezogen.

Dann kann eine Sehne 70, die von dem Schulterknochen 64 abgerissen ist, mit den Fadenenden 54, 56 und der Schlaufe 52 wieder am Schulterknochen 64 fixiert werden.

## Patentansprüche

1. Vorrichtung zum Einbringen eines Ankerelements, in dem ein Faden (38) eingefädelt ist, in einen Knochen (64) zum Fixieren eines vom Knochen abgelösten Gewebes, mit einem langerstreckten Körper (12), dessen distales Ende (18) derart ausgebildet ist, dass es mit dem Ankerelement (40) drehschlüssig verbindbar ist, wobei ein distaler Endabschnitt (22) des Körpers (12) mit zumindest einer Aussparung (24) versehen ist, in der ein fadenumkehrbildendes Ende einer Schlaufe (52) des Fadens (38) quer einlegbar ist, wobei die Aussparung (24) derart ausgebildet ist, dass die eingelegte Schlaufe (52) vor seitlichem Abfallen gehindert ist, und wobei im Bereich der Aussparung (24) in dem Körper (12) eine sich in Längsrichtung des Körpers (12) erstreckende und als durch den Körper (12) hindurchgehende Öffnung (34) ausgebildete Ausnehmung vorhanden ist, die die Aussparung (24) schneidet, **dadurch gekennzeichnet, dass** die Aussparung (24) als ein sich quer zur Öffnung (34) erstreckender Einschnitt (28) ausgebildet ist, so dass ein Fadenabschnitt (62) des fadenumkehrbildenden Schlaufenendes (52), der in den Einschnitt (28) eingelegt ist, freiliegend die Ausnehmung (32) überquert und daher von der Außenseite ergreifbar ist.

2. Vorrichtung nach Anspruch 1, **dadurch gekennzeichnet, dass** der Einschnitt (28), von proximal nach distal gesehen, nach distal geneigt verläuft.

3. Vorrichtung nach Anspruch 2, **dadurch gekennzeichnet, dass** der Einschnitt (28) mit einer Neigung im Bereich von 30° bis 90°, vorzugsweise von etwa 45°, verläuft.

4. Vorrichtung nach einem der Ansprüche 1 bis 3, **dadurch gekennzeichnet, dass** sich der Einschnitt (28) umfänglich, vorzugsweise halbumfänglich, um den Körper (12) erstreckt.

5. Vorrichtung nach einem der Ansprüche 1 bis 4, **dadurch gekennzeichnet, dass** der Einschnitt (28) in radialer Richtung eine Tiefe aufweist, die maximal der Hälfte des Durchmessers des Körpers (12) entspricht

6. Vorrichtung nach einem der Ansprüche 1 bis 5, **dadurch gekennzeichnet, dass** die Aussparung (24) als Schlitz (30) ausgebildet ist.

7. Vorrichtung nach einem der Ansprüche 1 bis 6, **dadurch gekennzeichnet, dass** sich Ausnehmung (32) und Einschnitt (28) mittig schneiden.

## Claims

1. Device for introducing an anchor element, in which a thread (38) is threaded in, into a bone (64) in order to fix a tissue that is detached from the bone, having an elongate body (12) whose distal end (18) is designed in such a way that it can be connected to the anchor element (40) in a rotationally locked manner, wherein a distal end portion (22) of the body (12) is provided with at least one recess (24) in which a turn-back end of a loop (52) of the thread (38) can be inserted crosswise, wherein the recess (24) is designed in that the inserted loop (52) is prevented from falling off to the side, and wherein a clearance designed as an opening (34) is provided in the area of the recess (24) within the body (12), which opening (34) extends in a longitudinal direction of the body (12) and which entirely passes the body (12) and which cuts the recess (24), **characterized in that** the recess (24) is designed as a cutting (28) extending crosswise to the opening (34), so that a thread section (62) of the turn-back end of the loop (52) which is inserted into the cutting (28) crosses the clearance (32) barely and can therefore be gripped from the outside.

2. Device of claim 1, **characterized in that** the cutting (28), seen from proximal to distal, extends with an inclination in the distal direction.

3. Device of claim 2, **characterized in that** the cutting (28) extends with an inclination in the range of 30° to 90°, preferably approximately 45°.

4. Device of anyone of claims 1 through 3, **characterized in that** the cutting (28) extends around the circumference of the body (12), preferably around half the circumference.

5. Device of anyone of claims 1 through 4, **characterized in that** the cutting (28), seen in a radial direction, has a depth that corresponds at most to half the diameter of the body (12).

6. Device of anyone of claims 1 through 5, **characterized in that** the recess (24) is designed as a slit (30).

7. Device of anyone of claims 1 through 6, **characterized in that** the clearance (32) and the cutting (28) intersect centrally.

## Revendications

1. Dispositif conçu pour insérer, dans un os (64), un élément d'ancrage dans lequel un fil (38) est intégré, en vue de la consignation à demeure d'un tissu détaché dudit os, comprenant un corps longiligne (12) dont l'extrémité distale (18) est réalisée de manière à pouvoir être reliée audit élément d'ancrage (40) avec solidarisation tournante, un tronçon extrême distal (22) dudit corps (12) étant pourvu d'au moins une dépouille (24) dans laquelle un bout de retour du file d'une boucle (52) dudit fil (38) peut être introduite transversalement, ladite dépouille (24) étant conçue pour empêcher une chute latérale de la boucle (52) introduite, sachant qu'un évidement, réalisé sous la forme d'un orifice (34) traversant le corps (12) de part en part, s'étend dans la direction longitudinale dudit corps (12) dans la région de la dépouille (24) pratiquée dans ledit corps (12), et croise ladite dépouille (24), **caractérisé par le fait que** la dépouille (24) revêt la forme d'une entaille (28) de telle sorte qu'un segment (62) du bout de retour du fil de la boucle (52), dépassant d'un côté d'un élément d'ancrage (40) et introduite dans ladite entaille (28), franchisse librement l'évidement (32) dans le sens transversal et puisse, de ce fait, être saisi depuis la face extérieure.

2. Dispositif selon la revendication 1, **caractérisé par le fait que** l'entaille (28), observée du côté proximal vers le côté distal, s'étend avec inclinaison vers ledit côté distal.

3. Dispositif selon la revendication 2, **caractérisé par le fait que** l'entaille (28) s'étend avec une inclinaison dans la plage respective de 30° à 90°, de préférence d'environ 45°.

4. Dispositif selon l'une des revendications 1 à 3, **caractérisé par le fait que** l'entaille (28) s'étend autour du corps (12) sur le périmètre, de préférence sur le demi-périmètre.

5. Dispositif selon l'une des revendications 1 à 4, **caractérisé par le fait que** l'entaille (28) présente, dans la direction radiale, une profondeur correspondant au maximum à la moitié du diamètre du corps (12).

6. Dispositif selon l'une des revendications 1 à 5, **caractérisé par le fait que** la dépouille (24) est réalisée sous la forme d'une fente (30).

7. Dispositif selon l'une des revendications 1 à 6, **caractérisé par le fait que** l'évidement (32) et l'entaille (28) s'entrecroisent centralement.
